(19)

Europäisches Patentamt
European Patent Office
Office européen des brevets

(11) **EP 3 819 639 B1**

(12) **EUROPEAN PATENT SPECIFICATION**

(45) Date of publication and mention
of the grant of the patent:
**08.11.2023 Bulletin 2023/45**

(51) International Patent Classification (IPC):
**G01N 33/574** (2006.01)     **G01N 27/62** (2021.01)
**G01N 33/53** (2006.01)

(21) Application number: **19834011.9**

(52) Cooperative Patent Classification (CPC):
**G01N 33/57434;** G01N 2333/96455;
G01N 2400/38; G01N 2800/56

(22) Date of filing: **05.07.2019**

(86) International application number:
**PCT/JP2019/026858**

(87) International publication number:
**WO 2020/013097 (16.01.2020 Gazette 2020/03)**

(54) **SUGAR CHAIN SPECIFIC TO PROSTATE CANCER, AND TEST METHOD USING SAME**

FÜR PROSTATAKREBS SPEZIFISCHE ZUCKERKETTE UND TESTVERFAHREN DAMIT

CHAÎNE DE SUCRE SPÉCIFIQUE DU CANCER DE LA PROSTATE ET PROCÉDÉ DE TEST L'UTILISANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.07.2018 JP 2018131774**

(43) Date of publication of application:
**12.05.2021 Bulletin 2021/19**

(73) Proprietor: **Japanese Foundation For Cancer
Research
Tokyo 135-8550 (JP)**

(72) Inventors:
• **UEDA Koji
Tokyo 135-8550 (JP)**
• **HAGA Yoshimi
Tokyo 135-8550 (JP)**

(74) Representative: **Prüfer & Partner mbB
Patentanwälte · Rechtsanwälte
Sohnckestraße 12
81479 München (DE)**

• **CHIKARA OHYAMA ET AL: "Carbohydrate
structure and differential binding of prostate
specific antigen to Maackia amurensis lectin
between prostate cancer and benign prostate
hypertrophy", GLYCOBIOLOGY, OXFORD
UNIVERSITY PRESS, US, vol. 14, no. 8, 24 March
2004 (2004-03-24) , pages 671-679, XP008139724,
ISSN: 0959-6658, DOI: 10.1093/GLYCOB/CWH071**
• **GUINEVERE S. M. KAMMEIJER ET AL: "An
In-Depth Glycosylation Assay for Urinary
Prostate-Specific Antigen", ANALYTICAL
CHEMISTRY, vol. 90, no. 7, 4 March 2018
(2018-03-04), pages 4414-4421, XP055675609, US
ISSN: 0003-2700, DOI:
10.1021/acs.analchem.7b04281**
• **KAZUHISA HAGIWARA ET AL: "Wisteria
floribunda Agglutinin and Its
Reactive-Glycan-Carrying Prostate-Specific
Antigen as a Novel Diagnostic and Prognostic
Marker of Prostate Cancer", INTERNATIONAL
JOURNAL OF MOLECULAR SCIENCES, vol. 18,
no. 2, 26 January 2017 (2017-01-26), page 261,
XP055483792, DOI: 10.3390/ijms18020261**

(56) References cited:
**WO-A1-2017/138457     WO-A1-2018/101328
JP-A- 2014 066 704     JP-A- 2015 078 972**

- **TOMOKAZU ISHIKAWA ET AL: "An Automated Micro-Total Immunoassay System for Measuring Cancer-Associated [alpha]2,3-linked Sialyl N-Glycan-Carrying Prostate-Specific Antigen May Improve the Accuracy of Prostate Cancer Diagnosis", INTERNATIONAL JOURNAL OF MOLECULAR SCIENCES, vol. 18, no. 2, 22 February 2017 (2017-02-22), page 470, XP055503642, DOI: 10.3390/ijms18020470**
- **KAMMEIJER, G.S.M. et al.: "An In-Depth Glycosylation Assay for Urinary Prostate-Specific Antigen", ANALYTICAL CHEMISTRY, vol. 90, no. 7, 4 March 2018 (2018-03-04), pages 4414-4421, XP055675609, ISSN: 0003-2700, DOI: 10.1021/acs.analchem.7b04281**
- **TAJIRI, M. et al.: "Oligosaccharide profiles of the prostate specific antigen in free and complexed forms from the prostate cancer patient serum and in seminal plasma: a glycopeptide approach", GLYCOBIOLOGY, vol. 18, no. 1, 23 October 2007 (2007-10-23), pages 2-8, XP055005162, DOI: 10.1093/glycob/cwm117**

**Description**

Technical Field

The present invention relates to a test method of prostate cancer diagnosis and the use of a test kit for prostate cancer.

Background Art

Prostate cancer is a cancer specific for men and frequently occurred in the elderly, and about 90% or more of the patients are 60 years or older. With the increase of the elderly, the number of the patients is increasing. According to the projected cancer cases by part in male in 2017 in the "Cancer Registry and Statistics" by Cancer Information Service, National Cancer Center, the number of patients newly suffering from prostate cancer is 86,100, ranking third in the number of cases following stomach and lung cancers, and is predicted to increase further with aging in the future. Prostate cancer progresses relatively slowly in many cases, and therapies such as radiation therapy and endocrine (hormone) therapy, in addition to surgery, can be taken as effective measures. Thus, prostate cancer is treatable if detected early.

Prostate cancer has been diagnosed by measuring prostate specific antigen (PSA) in blood. PSA screening is a blood test, thus it is minimally invasive, and has high sensitivity. PSA screening has thus been incorporated and performed in medical checkup as a test for prostate cancer.

However, PSA is produced in prostate epithelium, and is overproduced in not only prostate cancer, but also other prostate diseases such as prostatic hypertrophy and prostatitis. Thus, false positives often occur in patient screenings with PSA, which is considered a problem. PSA has a cutoff value of 4 ng/ml. When PSA value in blood is greater than or equal to the value, prostate cancer is suspected, and diagnosis by transrectal prostate palpation, transrectal echo, or transperineal prostate needle biopsy becomes necessary. For the range referred to as a "gray zone" with a PSA value of 4 to 10 ng/ml, there is a result that about 70% of the subjects do not suffer from cancer, and 50% of the subjects do not suffer from cancer even their PSA values are 10 to 20 ng/ml (Non Patent Literature 1).

When the diagnosis is determined by transperineal prostate needle biopsy, it is necessary to perform 10 to 12 tissue samplings in the first biopsy after hospitalization and anesthesia, and it is accompanied with severe pain. Partly because of the high false positive rate, many patients with PSA values in the range of the gray zone do not have prostate biopsies even when they are recommended for secondary examination in medical checkup, so they often miss opportunities for early detection. In fact, there are statistics showing that only about 30 to 50% of those who were determined to be PSA positive at medical checkup centers receive prostate biopsy.

Conventionally, methods that increase specificity of PSA test, distinguish prostate cancer from prostatic hypertrophy and specifically detect prostate cancer have been proposed (Non Patent Literatures 2 to 4, Patent Literatures 1 to 8). As PSA tests, Food and Drug Administration (FDA) has already approved prostate health index (PHI) (Non Patent Literature 2) and prostate cancer antigen 3 (PCA3) examination (Non Patent Literatures 3, 4). Patent Literatures 1 to 5 describe methods of contacting PSA with lectin that recognizes specific binding sialic acids, and performing examination using the binding properties with lectin. Patent Literatures 6 to 8 disclose methods of analyzing sugar chain structures by mass spectrometry to detect prostate cancer. Non Patent Literature 9 analyzes the structure and relative amounts of prostate specific antigen (PSA) in semen by mass spectrometry (MALDI-TOF MS). Non Patent Literature 10 discloses the establishing of a high-performance PSA Glycomics Assay (PGA) allowing the differentiation of $\alpha$2,6- and $\alpha$2,3-sialylated isomers. Non Patent Literature 11 discloses that the *Wisteria floribunda* agglutinin (WFA)-reactivity on prostate tumor is an independent risk factor of PSA recurrence. Non Patent Literature 12 examined whether the serum PCa (prostate cancer)-associated $\alpha$2,3-linked sialyl N-glycan carrying PSA (S2,3PSA) ratio measured by automated micro-total immunoassay systems ($\mu$TAS system) can be applied as a diagnostic marker of PCa. It is speculated that the $\mu$TAS system may improve the accuracy of PCa diagnosis.

Citation List

Patent Literature

Patent Literature 1: Japanese Patent Laid-Open No. 2002-55108
Patent Literature 2: Japanese Patent Laid-Open No. 2010-91308
Patent Literature 3: Japanese Translation of PCT International Application Publication No. 2011-529184
Patent Literature 4: Japanese Patent Laid-Open No. 2013-076666

Patent Literature 5: International Publication No. WO 2010/090264
Patent Literature 6: International Publication No. WO 2009/008381
Patent Literature 7: International Publication No. WO 2010/064683
Patent Literature 8: Japanese Patent Laid-Open No. 2011-137754
Patent Literature 9: Japanese Patent Laid-Open No. 2014-066704

Non Patent Literature

Non Patent Literature 1: Catalona WJ., et al., 1998, Jama, Vol.279, pp.1542-1547
Non Patent Literature 2: Loeb, S. et al., 2015, J. Urol. Vol.193, pp.1163-1169.
Non Patent Literature 3: Wei., J.T. et al., 2014, J. Clin. Oncol., Vol.32, pp.4066-4072.
Non Patent Literature 4: Schmid, M. et al., 2015, Advances in experimental medicine and biology, Vol. 867, pp.277-89
Non Patent Literature 5: Wang, W. et al., 2014, Sci. Rep. Vol.4, p.5012
Non Patent Literature 6: Vlaeminck-Guillem V. et al., 2010, Urology. Vol.75, pp.447-453.
Non Patent Literature 7: Wada, Y. 2013, Methods in molecular biology (Clifton, NJ), Vol.951, pp.245-253. Non Patent Literature 8: Toyama, A. et al., 2012, Anal. Chem. Vol.84, pp.9655-9662
Non Patent Literature 9: Ohyama et al., 2004, Glycobiology, Oxford University Press, US, Vol.14, pp. 671-679
Non Patent Literature 10: Kammeijer et al., 2018, Analytical Chemistry Vol.90, pp. 4414-4421
Non Patent Literature 11: Hagiwara et al., 2017, International Journal of Molecular Sciences, Vol.18, p. 261
Non Patent Literature 12: Ishikawa et al., 2017, International Journal of Molecular Sciences, Vol.18, p. 470

Summary of Invention

Technical Problem

All of the examination methods disclosed in the above literatures are, however, problematic in specificity or sensitivity, and any of them failed to specifically identify prostate cancer. PHI is high in sensitivity of 85%, but low in specificity of 45%, and cannot specifically identify prostate cancer (Non Patent Literature 5). PCA3 is an excellent examination method in that it is not affected by diseases other than cancer such as prostatic hypertrophy and prostatitis, but the results of four clinical tests showed that it is low in sensitivity, which is 53 to 84%, and insufficient to detect prostate cancer in the patients having PSA values of the gray zone (Non Patent Literature 6).

In the methods described in Patent Literatures 1 to 5, prostate cancer is diagnosed by analyzing saccharides modifying PSA by the binding properties to lectin. However, a problem is that lectin has low affinity compared to antibodies, leading to low sensitivity. Another problem is that lectin does not detect only saccharides modifying target PSA, and also binds to saccharides modifying other proteins. Thus, the methods using lectin cannot completely eliminate false positives.

Patent Literatures 6 to 8 disclose methods of identifying prostate cancer and prostatic hypertrophy by mass spectrometry. Patent Literature 6 describes a method for detecting prostate cancer by a ratio of fucose-bound sugar chains to fucose-unbound sugar chains, Patent Literature 7 describes that by an amount of sugar chains having LacdiNAc, and Patent Literature 8 describes that by the presence or absence of three or more sugar chains having LacdiNAc. However, none of them were enough to eliminate false positives because they are low in specificity.

PSA has been modified by sugar chains, and it has been reported that the type of modifying saccharides is correlates with prostate disease. However, accurate distinguishing between prostate cancer and prostatic hypertrophy has not achieved yet. An object of the present invention is to provide a method for accurately detecting prostate cancer even in a range of the gray zone in which a slight increase in PSA is observed, and to provide an index for identifying prostate cancer.

Solution to Problem

The present invention relates to the following method for testing prostate cancer and use of a test kit for prostate cancer. Furthermore, it relates to the use of a test kit for prostate cancer.

(1) A test method of prostate cancer, comprising: analyzing a sugar chain modifying PSA in a specimen; and analyzing a multisialylated LacdiNAc structure.

(2) The test method of prostate cancer according to (1), comprising:

analyzing a sugar chain modifying PSA in a specimen; and
analyzing relative abundance(s) of
[HexNAc]$_7$[Hexose]$_5$[Fucose][Neu5Ac]$_2$,
[HexNAc]$_7$[Hexose]$_6$[Neu5Ac]$_3$,
[HexNAc]$_7$[Hexose]$_6$[Fucose][Neu5Ac]$_2$, and/or
[HexNAc]$_7$[Hexose]$_6$[Fucose][NeuSAc]$_3$.

(3) The test method of prostate cancer according to (2), wherein the relative abundances of

[HexNAc]$_7$[Hexose]$_5$[Fucose][Neu5Ac]$_2$ and
[HexNAc]$_7$[Hexose]$_6$[NeuSAc]$_3$ are analyzed by logistic analysis.

(4) The test method of prostate cancer according to (2), comprising:

substituting the relative abundances of
[HexNAc]$_7$[Hexose]$_5$[Fucose][Neu5Ac]$_2$ and
[HexNAc]$_7$[Hexose]$_6$[Neu5Ac]$_3$ into the following formula 1:
[Expression 1]

$$\log_e(p/(1-p)) = -5.85 + 4.72x_1 + 0.80x_2 \qquad \text{(Formula 1)}$$

wherein p represents a value of PSA G-index; $x_1$ represents a relative abundance of [HexNAc]$_7$[Hexose]$_5$[Fucose][Neu5Ac]$_2$, and $x_2$ represents a relative abundance of [HexNAc]$_7$[Hexose]$_6$[Neu5Ac]$_3$.

(5) The test method of prostate cancer according to any one of (1) to (4), wherein the specimen is blood, serum, plasma, or urine.

(6) The test method of prostate cancer according to any one of (1) to (5), wherein the specimen is a specimen obtained from a patient having a PSA value of 4 to 10 ng/ml.

(7) The test method of prostate cancer according to any one of (1) to (6), wherein the sugar chain is analyzed using an oxonium monitoring method.

(8) The test method of prostate cancer according to (1), (2), (5) or (6), wherein the analysis uses an antibody or lectin.

(9) The test method of prostate cancer according to (8), wherein the method uses an antibody or lectin that specifically recognizes a saccharide having a multisialylated LacdiNAc structure.

(10) Use of a test kit for prostate cancer, comprising lectin that specifically recognizes a saccharide having a multisialylated LacdiNAc structure, wherein the use is for a method according to any one of (1) to (9).

(11) Use of the test kit according to (10), wherein the saccharide having a multisialylated LacdiNAc structure is

[HexNAc]$_7$[Hexose]$_5$[Fucose][Neu5Ac]$_2$,
[HexNAc]$_7$[Hexose]$_6$[NeuSAc]$_3$, [HexNAc]$_3$[Hexose]$_4$[Neu5Ac], or [HexNAc]$_5$[Hexose]$_6$[Neu5Ac]$_2$.

Brief Description of Drawings

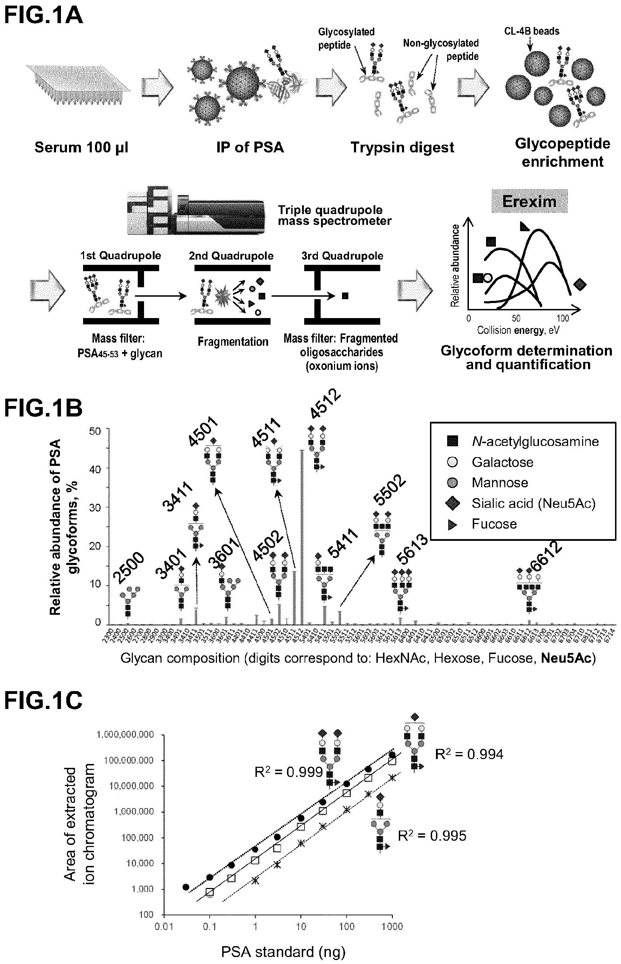

[Figure 1] Figure 1 is a diagram showing sugar chain structure analysis of PSA using an oxonium monitoring method. Figure 1A shows an overview of the oxonium monitoring method. Figures 1B and 1C show the analysis results with PSA standard.

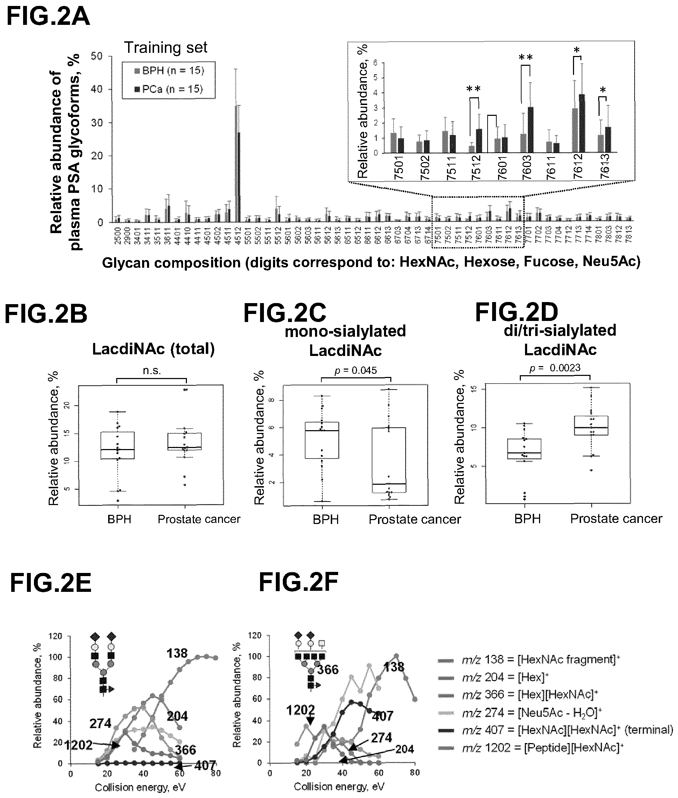

[Figure 2] Figure 2 is a diagram showing sugar chain analysis of PSA clinical samples. Figure 2A shows types and abundance of sugar chains detected in a prostatic hypertrophy patient group and a prostate cancer patient group of a training set. Figures 2B to 2D show relative abundances of sugar chains having a LacdiNAc structure detected in the prostatic hypertrophy patient group and the prostate cancer patient group. Figures 2E and 2F show the results of Erexim(R) analysis of representative sugar chains modifying PSA in serum of prostate cancer patients.

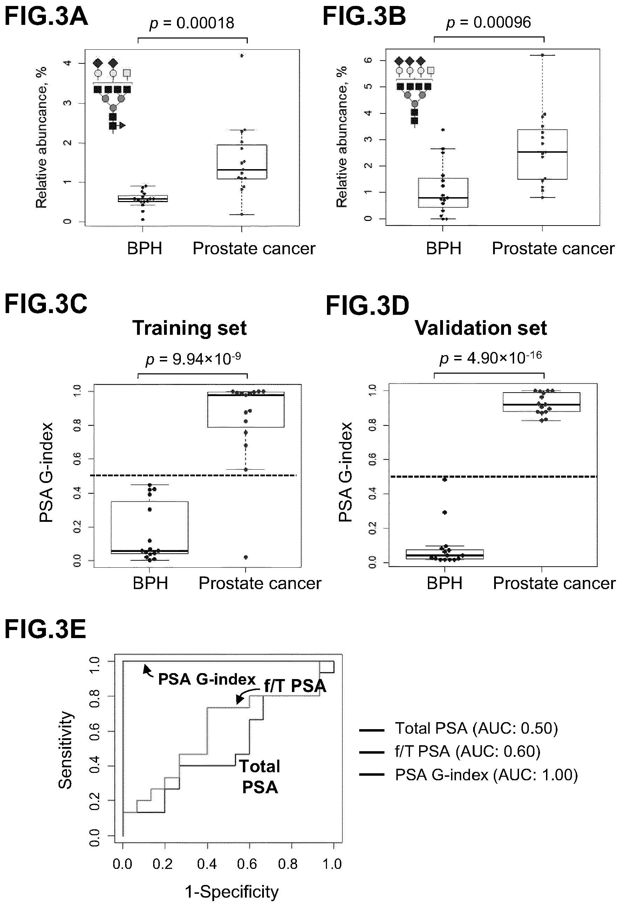

[Figure 3] Figure 3 is a diagram showing the results with PSA G-index. Figure 3A shows relative abundances of Glycan ID: 7512 in a prostatic hypertrophy patient group and a prostate cancer patient group and Figure 3B shows relative abundances of Glycan ID: 7603 in the prostatic hypertrophy patient group and the prostate cancer patient group. Figure 3C shows a plot of PSA G-index values for the prostatic hypertrophy patient group and the prostate cancer patient group of a training set. Figure 3D shows a plot of PSA G-index values for the prostatic hypertrophy patient group and the prostate cancer patient group of a validation set. Figure 3E shows ROC curves with PSA, PSA f/T, and PSA G-index values. It should be noted that Glycan ID defines the type of glycan, and the digits correspond to the numbers of HexNAc, Hexose, Fucose, and Neu5Ac from the left, respectively, which are described later in detail.

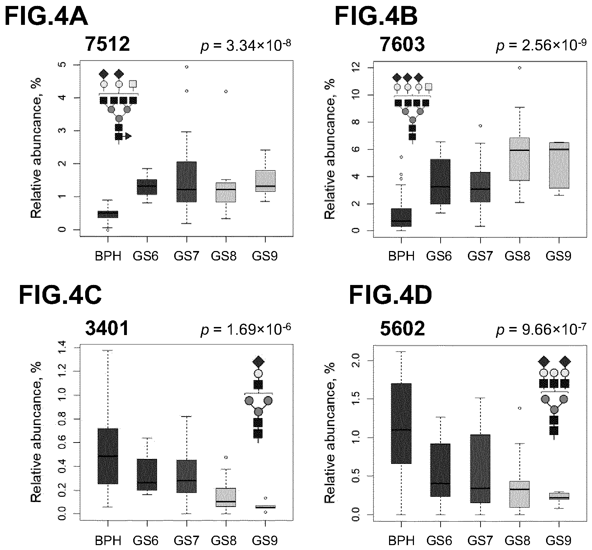

[Figure 4] Figure 4 is a diagram showing sugar chains capable of classifying the grade (malignancy) of prostate cancer. Figures 4A, 4B, 4C, and 4D show correlations of Glycan ID: 7512, Glycan ID: 7603, Glycan ID: 3401, and Glycan ID: 5602, with a Gleason score, respectively.

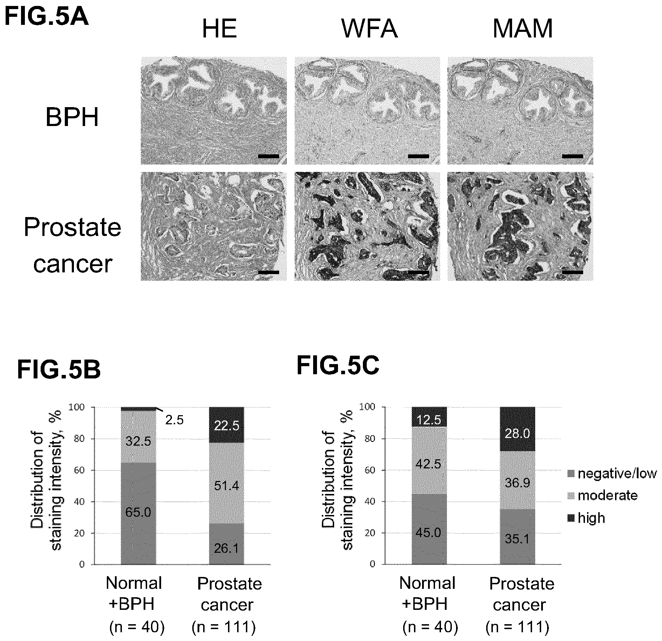

[Figure 5] Figure 5 is a diagram showing the analysis results by lectin tissue staining. Figure 5A shows prostate tissue staining results with HE, WFA, and MAM in prostatic hypertrophy patients and prostate cancer patients. Figure 5B shows prostate tissue staining intensity with WFA in prostatic hypertrophy patients and prostate cancer patients. Figure 5C shows prostate tissue staining intensity with MAM in prostatic hypertrophy patients and prostate cancer patients.

Description of Embodiments

The analysis by the present inventors has revealed that there is a significant increase in certain sugar chains having LacdiNAc (GalNAc$\beta$1-4GlcNAc, N-acetylgalactosamine-N-acetylglucosamine) structure in patients suffering from prostate cancer. The analysis has been performed by mass spectrometry, but any method can be used as long as it is possible to recognize or analyze the multisialylated LacdiNAc structure or the specific sugar chain indicated by Glycan ID below.

The analytical method by mass spectrometry is a method that can be examined with great sensitivity as shown below, but not limiting to mass spectrometry, the analysis can be performed using anything that recognizes a particular sugar chain, such as an antibody, lectin, or aptamer which recognizes a multisialylated LacdiNAc structure. When the detection is performed with an antibody, the detection may be performed by any method used in the art, such as ELISA or SPR. When the detection is performed with a lectin, the detection may be performed by any method such as lectin blot or capillary electrophoresis. As shown in the Examples below, lectins such as WFA or MAM does not specifically recognize a multisialylated LacdiNAc structure. However, a system capable of specifically detecting a multisialylated LacdiNAc structure can be created by using several lectins in combination.

The present invention can be also carried out not only by analysis of relative abundances of Glycan ID: 7512 and Glycan ID: 7603, but also by profile recognition on profiling data of saccharides containing a multisialylated LacdiNAc structure. Specifically, profiles of multiple sugar chains obtained from patients having diagnosis determined prostate cancer are machine-learned by a computer as training data, and used it as a diagnostic support system. Then, during the test, by inputting the profile obtained from the subject as it is, prostate cancer candidate data may be detected by pattern recognition.

As shown in the Examples below, as a result of analyzing sugar chain structures modifying PSA, it has been revealed that prostate cancer can be detected with high accuracy by using PSA G-index defined below. Examination with PSA G-index on patients of a gray zone in conventional PSA tests as a secondary screening makes it possible to detect patients suffering from prostate cancer in a non-invasive manner. Further details are described below with showing data.

First, whether sugar chains modifying PSA are able to be sensitively detected and quantified was confirmed with PSA standard. PSA obtained from human semen was dissolved in 8 M urea, 50 mM HEPES-NaOH, pH 8.0, reduced with 10 mM DTT (GE Healthcare Life Science), alkylated with 25 mM iodoacetamide (Sigma-Aldrich), and then digested with Trypsin/Lys-C mix (Promega Corporation). Glycoproteins were enriched by hydrophilic purification method (Non Patent Literature 7), and dried in vacuo.

The resulting glycoproteins were analyzed by oxonium ion monitoring method (Erexim method, Non Patent Literature 8, Patent Literature 9) with a triple quadrupole mass spectrometer LCMS-8060 (Shimadzu Corporation) coupled with a Prominence nanoflow liquid chromatogram to identify sugar chains.

Figure 1A schematically shows an overview of the oxonium ion monitoring method applied by multiple-reaction monitoring mass spectrometry (MRM-MS). PSA is degraded with trypsin into a mixture of peptides and glycopeptides, and they are separated with a nanoflow liquid chromatogram. In a mass spectrometer, glycopeptide ions having a particular mass are isolated at the first quadrupole (Q1). The isolated glycopeptide ions are introduced into the second quadrupole (Q2), and cause collision induced dissociation (CID). In the third quadrupole (Q3), oxonium ions from saccharides are selectively detected with a filter. Under optimal collision energy conditions, oxonium ions of m/z 138.1 function as quantitative reporters of glycopeptides of various saccharide compositions.

The results of performing sugar chain structure analysis with 100 ng of PSA standard obtained from human semen are shown in Figure 1B. The digits on the horizontal axis in the figure define the type of each glycan as Glycan ID, and correspond to the numbers of HexNAc, Hexose, Fucose, and Neu5Ac from the left, respectively. The type and quantitative distribution of saccharides modifying asparagine at position 45 of PSA were analyzed. As a result, it was revealed by oxonium ion monitoring method that there were 67 types of saccharide structures.

Oxonium ion monitoring method has been found to be not only a method with which the analysis was completed in a short time of 25 minutes of LS/MS operation time without the need for enzymatic separation of glycans or chemical modification, but also a very sensitive detection method. The saccharide of the highest content was 4[HexNAc]5[Hex]1[Fuc]2[Neu5Ac] (Glycan ID: 4512, 44.5 $\pm$ 0.9%), and the saccharide of the lowest content was 6[Hex]7[NAcHex]0[Fuc]0[Neu5Ac] (Glycan ID: 6700, 0.01 + 0.001%). There has been previously no report of detecting such a very large number of types of saccharides as 67 types of glycans modifying PSA, or detecting a very small amount of saccharide as 0.01%. Thus, it has been shown that oxonium ion monitoring method is a very sensitive method.

Figure 1C shows the dynamic range of PSA sugar chain analysis. PSA digested with trypsin was serially diluted, and the detection limit was analyzed. As a result, it was possible to detect up to 1 fmol of the glycopeptide obtained from 0.03 ng of PSA, and the quantitative dynamic range was 5 digits or more ($R^2 > 0.99$). This result indicates that sugar chain analysis by oxonium ion monitoring method can perform test sufficiently even with PSA in a gray zone of 4 to 10 ng/ml.

Next, sugar chain analysis of PSA was performed using samples obtained from 15 prostate cancer patients and 15 prostatic hypertrophy patients having a PSA value in a gray zone, 4 to 10 ng/ml, to determine an index identifying prostate cancer, as a training set. Table 1 shows patient characteristics in the training set.

Analysis with clinical samples uses serum samples of prostate cancer patients and prostatic hypertrophy patients. It should be noted that final diagnosis is determined from histopathological diagnosis by prostate biopsy. Purification of PSA was performed as follows. To the serum was added 4-fold amount of wash solution (0.1% Tween-20 in PBS). The obtained solution was mixed with Protein G Sepharose to which anti-PSA monoclonal antibodies were immobilized (Fitzgerald), reacted overnight at 4°C, and washed. Then, PSA was eluted with 8 M urea, 50 mM HEPES-NaOH, pH 8.0, and purified. The eluted protein was reduced with 10 mM DTT, alkylated with 25 mM iodoacetamide, and then digested with Trypsin/Lys-C mix. Glycoproteins were enriched by hydrophilic purification method (Non Patent Literature 7), and dried in vacuo. It should be noted that, although serum samples were used here, not only blood derived samples but also urine can be used as samples.

[Table 1]

| Characteristics | Training set | | Validation sample set | |
|---|---|---|---|---|
| | Prostate cancer | Prostatic hypertrophy | Prostate cancer | Prostatic hypertrophy |
| Number of patients | 15 | 15 | 15 | 15 |
| Age, Mean $\pm$ SD (range) | 69.1 $\pm$ 6.69 (53-79) | 68.1 $\pm$ 5.0 (60-79) | 70.3 $\pm$ 3.3 (65-75) | 69.9 $\pm$ 6.4 (58-79) |
| PSA (ng/mL) Mean $\pm$ SD (range) | 7.5 $\pm$ 1.6 (4.19-9.76) | 7.4 $\pm$ 1.5 (4.31-9.36) | 7.5 $\pm$ 1.5 (5.41-9.67) | 7.5 $\pm$ 1.4 (5.32-9.43) |
| f/T PSA (%) Mean $\pm$ SD (range) | 16.0 $\pm$ 6.4 (8.4-34.2) | 20.8 $\pm$ 7.7 (9.8-45.1) | 16.1 $\pm$ 5.9 (9.2-27.8) | 19.3 $\pm$ 7.5 (9.8-34.0) |

In the same manner as described above, 52 sugar chain structures on PSA could be quantified (Figure 2A). Sugar chain structures of Glycan ID: 7512, 7603, 7612, and 7613 showed significant quantitative differences between the prostatic hypertrophy patient group and the prostate cancer patient group. It has also been found that sugar chains modifying PSA differ between the prostate cancer patient group and the healthy subject group, although no data is shown here. Thus, it is possible to distinguish prostate cancer patients from others, namely patients suffering from prostate diseases other than prostate cancer and healthy subjects, by analyzing sugar chain structures.

Among the 52 types of sugar chains, sugar chains containing a multisialylated structure, specifically di-/trisialylated LacdiNAc (GalNAc$\beta$1-4GlcNAc), were significantly increased in the prostate cancer patient group compared to the prostatic hypertrophy patient group (Figure 2 (D), p = 0.0023). Meanwhile, the total amount of sialylated LacdiNAc (Figure 2B) or sugar chain structures to which mono-sialylated LacdiNAc is attached (Figure 2C) did not show significant changes between the prostatic hypertrophy patient group and the prostate cancer patient group. The terminal LacdiNAc structure and the presence of sialic acid (Neu5Ac) residues were also confirmed by Erexim method (Figures 2E and 2F).

Based on these findings, we aimed to establish a novel diagnostic algorithm that complements the specificity of PSA test and can reliably reduce false positive rates. Two sugar chain structures specific for prostate cancer, Glycan ID: 7512 (p = $9.91 \times 10^{-8}$) and Glycan ID: 7603 (p = $1.66 \times 10^{-5}$), which showed significant differences between the prostate cancer patient group and the prostatic hypertrophy patient group in the training set, were selected, and the relative abundance thereof were plotted. Figure 3A shows the relative abundances of Glycan ID: 7512 in prostatic hypertrophy patients (BPH) and prostate cancer patients, and Figure 3B shows the relative abundances of Glycan ID: 7603 in those. Both Glycan ID: 7512 and 7603 show significant difference in abundance between them.

Based on these results, a diagnostic model (PSA G-index) based on logistic regression was established. In formula 1, p represents a value of PSA G-index; $x_1$ represents a relative abundance of Glycan ID: 7512; and $x_2$ represents a relative abundance of Glycan ID: 7603.

[Expression 3]

$$\log_e(p/(1-p)) = -5.85 + 4.72x_1 + 0.80x_2$$

(Formula 1)

When a cutoff value of PSA G-index was set to 0.5, the sensitivity and specificity of the training set were 93.3% and 100%, respectively (Figure 3C). It should be noted that, since PSA G-index is based on logistic analysis, the formula may differ slightly depending on the increase in the number of specimens to be analyzed in the future. However, it is possible to identify prostate cancer and other prostate diseases with high sensitivity and specificity by detecting Glycan ID: 7512 and Glycan ID: 7603 and using their amounts for identification.

Although all the saccharides modifying PSA are analyzed and their relative amounts are determined here, analysis may be performed of only a particular saccharide, such as a saccharide having a multisialylated LacdiNAc structure. In addition, among the peptides of PSA, peptides in the region that is not glycosylated may be taken as a reference, and a ratio of PSA modified by a particular saccharide to total PSA may then be determined to use for analysis. Although the relative values of saccharides described above differ depending on the saccharide used as a reference or the amount of PSA, the amounts of the saccharides are significantly different in prostate cancer and other prostate diseases, and thus it is possible to distinguish prostate cancer and other prostate diseases with high sensitivity by logistic analysis.

The PSA G-index was then evaluated using the validation sample set of Table 1 (Figure 3D). All clinical samples were diagnosed as correct disease in both of the 15-case prostatic hypertrophy patient group and the 15-case prostate cancer patient group. In ROC curve analysis, the PSA G-index area under the curve (AUC) was 1.00 (100% sensitivity and 100% specificity), while AUCs of the PSA value (Total PSA) and the ratio of free PSA to total PSA (PSA f/T) were 0.50 (80.0% sensitivity, 33.3% specificity) and 0.60 (73.3% sensitivity, 60.0% specificity), respectively (Figure 3E). These results indicate that PSA G-index can significantly improve specificity of prostate cancer diagnosis and avoid false positives compared to PSA or PSA fiT values, which are conventionally indexes of prostate cancer.

Next, whether sugar chain structures on PSA in serum reflect the grade (malignancy) of prostate cancer was analyzed. Using serum from 77 patients of different grades shown in Table 2, sugar chain analysis by oxonium ion monitoring method was performed.

[Table 2]

| Characteristics | Prostatic hypertrophy | GS6 | GS7 | GS8 | GS9 |
|---|---|---|---|---|---|
| Number of patients (range) | 30 | 8 | 23 | 11 | 5 |
| Age, Mean ± SD (range) | 69.1 ± 5.8 (58-79) | 73.8 ± 3.4 (67-79) | 68.9 ± 5.2 (53-78) | 72.5 ± 4.5 (67-78) | 75.2 ± 6.2 (65-84) |
| PSA (ng/mL) Mean ± SD (range) | 7.4 ± 1.4 (4.31-9.43) | 9.7 ± 4.7 (5.57-21.14) | 10.7 ± 7.0 (5.5-27.85) | 14.9 ± 1.4 (5.41-28.67) | 50.8 ± 33.9 (6.88-107.54) |
| f/T PSA (%) Mean ± SD (range) | 20.1 ± 7.6 (9.8-45.1) | 19.5 ± 8.9 (6.3-34.2) | 14.2 ± 5.6 (7.0-26.7) | 14.8 ± 8.3 (9.3-34.9) | 11.7 ± 6.2 (7.3-24.1) |

As a result, the frequency of Glycan ID: 7512 (Figure 4A) or Glycan ID: 7603 (Figure 4B), which are sugar chain structure to which multiple sialylated LacdiNAc are attached, was positively correlated with Gleason score (GS), an index of malignancy by prostate biopsy (p = $3.34 \times 10^{-8}$, or p = $2.56 \times 10^{-9}$). The Gleason score is an index that classifies the malignancy of prostate cancer that occurs with different degrees of malignancy in the same prostate. The Gleason score is classified into 9 stages of GS2 to GS10 by the sum of the values obtained by determining predominant and ancillary lesions from biopsy. The higher the Gleason score value indicates the higher the malignancy.

Furthermore, Glycan ID: 3401 (Figure 4C) or Glycan ID: 5602 (Figure 4D) showed a negative correlation with the Gleason score (p = $1.69 \times 10^{-6}$, or p = $9.66 \times 10^{-7}$). These results indicate the possibility that the pathological malignancy of prostate cancer is correlated with the amounts of certain sugar chains, and the malignancy of prostate cancer can be diagnosed by liquid biopsy.

To identify the cause of the increase of multisialylated LacdiNAc structure in serum PSA, lectin histochemical staining was performed using a tissue microarray (US Biomax) containing 9, 31, and 111 prostate tissues of normal, prostatic hypertrophy patients, and prostate cancer patients, respectively. Histochemical staining was performed using WFA, a lectin that detects a LacdiNAc structure, or MAM, a lectin that detects a Siaα2-3Gal structure. Biotinylated lectins were used for both WFA and MAM, and the analysis was performed using a Vectastain Elite ABC kit (Vector Laboratories). As a result, cytoplasmic and protoplasmic membranes of cancer cells were significantly stained (Figure 5A). The staining intensity was classified into three levels, "high", "moderate", and "low", and the stained images of tissues of prostate cancer patients and those of normal and prostatic hypertrophy patients were compared. In the prostate cancer tissues, images of "high" in staining intensity were observed at a high frequency of 9.00-fold when stained with WFA and 2.24-fold when stained with MAM (Figures 5B, 5C).

WFA and MAM lectins specifically recognize GalNAc structures containing a LacdiNAc structure and a Neu5Acα2-3Gal structure, respectively. The above results indicate that prostate cancer tissue tends to strongly express glycoproteins containing LacdiNAc and Neu5Ac structures. These histological findings were consistent with the results of PSA sugar chain structure analysis by mass spectrometry. Accordingly, it is possible to determine prostate cancer, and even the stage of prostate cancer, by analyzing sugar chains of PSA in blood without performing tissue biopsy.

Industrial Applicability

Test using the diagnostic marker, PSA G-index, shown in the Examples, performed as a secondary screening of patients diagnosed as having a suspected prostate cancer from the PSA value, can identify prostate cancer with good specificity. The test can identify even prostate cancer at an early stage with good specificity, which makes it possible to lead to early treatment.

**Claims**

1.   A test method of prostate cancer, comprising:

   analyzing a sugar chain modifying PSA in a specimen; and
   analyzing a multisialylated LacdiNAc structure.

2.   The test method of prostate cancer according to claim 1, comprising:

   analyzing a sugar chain modifying PSA in a specimen; and
   analyzing relative abundance(s) of
   $[\text{HexNAc}]_7[\text{Hexose}]_5[\text{Fucose}][\text{Neu5Ac}]_2$,
   $[\text{HexNAc}]_7[\text{Hexose}]_6[\text{Neu5Ac}]_3$,
   $[\text{HexNAc}]_7[\text{Hexose}]_6[\text{Fucose}][\text{Neu5Ac}]_2$, and/or
   $[\text{HexNAc}]_7[\text{Hexose}]_6[\text{Fucose}][\text{Neu5Ac}]_3$-

3.   The test method of prostate cancer according to claim 2, wherein the relative abundances of

   $[\text{HexNAc}]_7[\text{Hexose}]_5[\text{Fucose}][\text{Neu5Ac}]_2$ and
   $[\text{HexNAc}]_7[\text{Hexose}]_6[\text{NeuSAc}]_3$ are analyzed by logistic analysis.

4.   The test method of prostate cancer according to claim 2, comprising:

   substituting the relative abundances of

[HexNAc]$_7$[Hexose]$_5$[Fucose][Neu5Ac]$_2$ and
[HexNAc]$_7$[Hexose]$_6$[NeuSAc]$_3$ into the following formula 1: [Expression 1]

$$\log_e(p/(1-p)) = -5.85 + 4.72x_1 + 0.80x_2 \qquad \text{(Formula 1)}$$

wherein p represents a value of PSA G-index; $x_1$ represents a relative abundance of [HexNAc]$_7$[Hexose]$_5$[Fucose][Neu5Ac]$_2$, and $x_2$ represents a relative abundance of
[HexNAc]$_7$[Hexose]$_6$[Neu5Ac]$_3$.

5. The test method of prostate cancer according to any one of claims 1 to 4, wherein the specimen is blood, serum, plasma, or urine.

6. The test method of prostate cancer according to any one of claims 1 to 5, wherein the specimen is a specimen obtained from a patient having a PSA value of 4 to 10 ng/ml.

7. The test method of prostate cancer according to any one of claims 1 to 6, wherein the sugar chain is analyzed using an oxonium monitoring method.

8. The test method of prostate cancer according to claim 1, 2, 5 or 6, wherein the analysis uses an antibody or lectin.

9. The test method of prostate cancer according to claim 8, wherein the method uses an antibody or lectin that specifically recognizes a saccharide having a multisialylated LacdiNAc structure.

10. Use of a test kit for prostate cancer, comprising lectin that specifically recognizes a saccharide having a multisialylated LacdiNAc structure, wherein the use is for a method according to any one of claims 1 to 9.

11. Use of the test kit according to claim 10, wherein the saccharide having a multisialylated LacdiNAc structure is

[HexNAc]$_7$[Hexose]$_5$[Fucose][Neu5Ac]$_2$,
[HexNAc]$_7$[Hexose]$_6$[Neu5Ac]$_3$, [HexNAc]$_3$[Hexose]$_4$[Neu5Ac], or
[HexNAc]$_5$[Hexose]$_6$[Neu5Ac]$_2$.

**Patentansprüche**

1. Testverfahren für Prostatakrebs, umfassend:

Analysieren einer Zuckerkette, die PSA in einer Probe modifiziert; und
Analysieren einer multisialylierten LacdiNAc-Struktur.

2. Testverfahren für Prostatakrebs nach Anspruch 1, umfassend:

Analysieren einer Zuckerkette, die PSA in einer Probe modifiziert; und
Analysieren der relativen Häufigkeit(en) von
[HexNAc]$_7$[Hexose]$_5$[Fucose][Neu5Ac]$_2$,
[HexNAc]$_7$[Hexose]$_6$[Neu5Ac]$_3$,
[HexNAc]$_7$[Hexose]$_6$[Fucose][Neu5Ac]$_2$, und/oder
[HexNAc]$_7$[Hexose]$_6$[Fucose][Neu5Ac]$_3$.

3. Testverfahren für Prostatakrebs nach Anspruch 2, wobei die relativen Häufigkeiten von

[HexNAc]$_7$[Hexose]$_5$[Fucose][Neu5Ac]$_2$ und
[HexNAc]$_7$[Hexose]$_6$[Neu5Ac]$_3$ durch logistische Analyse analysiert werden.

4. Testverfahren für Prostatakrebs nach Anspruch 2, unfassend:

Einsetzen der relativen Häufigkeiten von
[HexNAc]$_7$[Hexose]$_5$[Fucose][Neu5Ac]$_2$ und
[HexNAc]$_7$[Hexose]$_6$[Neu5Ac]$_3$ in die folgende Formel 1: [Ausdruck 1]

$$\log_e(p/(1-p)) = -5.85 + 4.72x_1 + 0.80x_2 \qquad \text{(Formel 1)}$$

wobei p einen Wert des PSA G-Index darstellt; $x_1$ eine relative Häufigkeit von [HexNAc]$_7$[Hexose]$_5$[Fucose][Neu5Ac]$_2$ darstellt, und $x_2$ eine relative Häufigkeit von [HexNAc]$_7$[Hexose]$_6$[Neu5Ac]$_3$ darstellt.

5. Testverfahren für Prostatakrebs nach irgendeinem der Ansprüche 1 bis 4, wobei die Probe Blut, Serum, Plasma oder Urin ist.

6. Testverfahren für Prostatakrebs nach irgendeinem der Ansprüche 1 bis 5, wobei die Probe eine Probe ist, die von einem Patienten mit einem PSA-Wert von 4 bis 10 ng/ml erhalten wurde.

7. Testverfahren für Prostatakrebs nach irgendeinem der Ansprüche 1 bis 6, wobei die Zuckerkette unter Verwendung eines Oxonium-Überwachungsverfahrens analysiert wird.

8. Testverfahren für Prostatakrebs nach Anspruch 1, 2, 5 oder 6, wobei für die Analyse ein Antikörper oder Lektin verwendet wird.

9. Testverfahren für Prostatakrebs nach Anspruch 8, wobei das Verfahren einen Antikörper oder ein Lektin verwendet, der/das spezifisch ein Saccharid mit einer multisialylierten LacdiNAc-Struktur erkennt.

10. Verwendung eines Testkits für Prostatakrebs, umfassend Lektin, das spezifisch ein Saccharid mit einer multisialylierten LacdiNAc-Struktur erkennt, wobei die Verwendung für ein Verfahren nach einem der Ansprüche 1 bis 9 erfolgt.

11. Verwendung des Testkits nach Anspruch 10, wobei das Saccharid mit einer multisialylierten LacdiNAc-Struktur

[HexNAc]$_7$[Hexose]$_5$[Fucose][Neu5Ac]$_2$,
[HexNAc]$_7$[Hexose]$_6$[Neu5Ac]$_3$, [HexNAc]$_3$[Hexose]$_4$[Neu5Ac],
oder [HexNAc]$_5$[Hexose]$_6$[Neu5Ac]$_2$ ist.

**Revendications**

1. Un procédé de test du cancer de la prostate, comprenant :

analyser un PSA modifiant la chaîne de sucre dans un échantillon ; et
analyser une structure LacdiNAc multisialylée.

2. Le procédé de test du cancer de la prostate selon la revendication 1, comprenant :

analyser un PSA modifiant la chaîne de sucre dans un échantillon ; et
analyser la ou les abondance(s) relative(s) de [HexNAc]$_7$[Hexose]$_5$[Fucose][Neu5Ac]$_2$,
[HexNAc]$_7$[Hexose]$_6$[Neu5Ac]$_3$,
[HexNAc]$_7$[Hexose]$_6$[Fucose][Neu5Ac]$_2$, et/ou
[HexNAc]$_7$[Hexose]$_6$[Fucose][Neu5Ac]$_3$.

3. Le procédé de test du cancer de la prostate selon la revendication 2, dans lequel les abondances relatives de

[HexNAc]$_7$[Hexose]$_5$[Fucose][Neu5Ac]$_2$ et
[HexNAc]$_7$[Hexose]$_6$[Neu5Ac]$_3$ sont analysées par analyse logistique.

4. Le procédé de test du cancer de la prostate selon la revendication 2, comprenant :

substituer les abondances relatives de
$[HexNAc]_7[Hexose]_5[Fucose][Neu5Ac]_2$ et
$[HexNAc]_7[Hexose]_6[Neu5Ac]_3$ dans la formule 1 suivante :
[Expression 1]

$$\log_e(p/(1-p)) = -5.85 + 4.72x_1 + 0.80x_2 \quad \text{(Formule 1)}$$

dans laquelle p représente une valeur de l'indice G du PSA ; $x_1$ représente une abondance relative de $[HexNAc]_7[Hexose]_5[Fucose][Neu5Ac]_2$, et $x_2$ représente une abondance relative de $[HexNAc]_7[Hexose]_6[Neu5Ac]_3$.

5. Le procédé de test du cancer de la prostate selon l'une quelconque des revendications 1 à 4, dans lequel l'échantillon est du sang, du sérum, du plasma ou de l'urine.

6. Le procédé de test du cancer de la prostate selon l'une quelconque des revendications 1 à 5, dans lequel l'échantillon est un échantillon obtenu à partir d'un patient ayant une valeur de PSA de 4 à 10 ng/ml.

7. Le procédé de test du cancer de la prostate selon l'une quelconque des revendications 1 à 6, dans lequel la chaîne de sucre est analysée en utilisant un procédé de surveillance oxonium.

8. Le procédé de test du cancer de la prostate selon la revendication 1, 2, 5 ou 6, dans lequel l'analyse utilise un anticorps ou une lectine.

9. Le procédé de test du cancer de la prostate selon la revendication 8, dans lequel le procédé utilise un anticorps ou une lectine qui reconnaît spécifiquement un saccharide ayant une structure LacdiNAc multisialylée.

10. Utilisation d'un kit de test pour le cancer de la prostate, comprenant une lectine qui reconnaît spécifiquement un saccharide ayant une structure LacdiNAc multisialylée, dans laquelle l'utilisation est pour un procédé selon l'une quelconque des revendications 1 à 9.

11. Utilisation du kit de test selon la revendication 10, dans laquelle le saccharide ayant une structure LacdiNAc multi-sialylée est

$[HexNAc]_7[Hexose]_5[Fucose][Neu5Ac]_2$,
$[HexNAc]_7[Hexose]_6[Neu5Ac]_3$, $[HexNAc]_3[Hexose]_4[Neu5Ac]$, ou
$[HexNAc]_5[Hexose]_6[Neu5Ac]_2$.

# FIG.1A

| Serum 100 µl | IP of PSA | Trypsin digest | Glycopeptide enrichment |

# FIG.1B

Glycan composition (digits correspond to: HexNAc, Hexose, Fucose, **Neu5Ac**)

# FIG.1C

PSA standard (ng)

# FIG.2A

Glycan composition (digits correspond to: HexNAc, Hexose, Fucose, Neu5Ac)

# FIG.2B

### LacdiNAc (total)

# FIG.2C
## mono-sialylated
### LacdiNAc

# FIG.2D
## di/tri-sialylated
### LacdiNAc

# FIG.2E

# FIG.2F

$m/z$ 138 = [HexNAc fragment]$^+$
$m/z$ 204 = [Hex]$^+$
$m/z$ 366 = [Hex][HexNAc]$^+$
$m/z$ 274 = [Neu5Ac - H$_2$O]$^+$
$m/z$ 407 = [HexNAc][HexNAc]$^+$ (terminal)
$m/z$ 1202 = [Peptide][HexNAc]$^+$

## FIG.3A

$p = 0.00018$

## FIG.3B

$p = 0.00096$

## FIG.3C

### Training set

$p = 9.94 \times 10^{-9}$

## FIG.3D

### Validation set

$p = 4.90 \times 10^{-16}$

## FIG.3E

— Total PSA (AUC: 0.50)
— f/T PSA (AUC: 0.60)
— PSA G-index (AUC: 1.00)

# FIG.4A

# FIG.4B

# FIG.4C

# FIG.4D

**FIG.5A**

HE  WFA  MAM

BPH

Prostate
cancer

**FIG.5B**    **FIG.5C**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002055108 A
- JP 2010091308 A
- JP 2011529184 W
- JP 2013076666 A
- WO 2010090264 A
- WO 2009008381 A
- WO 2010064683 A
- JP 2011137754 A
- JP 2014066704 A

### Non-patent literature cited in the description

- Cancer Registry and Statistics. Cancer Information Service, National Cancer Center, 2017
- **CATALONA WJ et al.** *Jama,* 1998, vol. 279, 1542-1547
- **LOEB, S. et al.** *J. Urol.,* 2015, vol. 193, 1163-1169
- **WEI., J.T. et al.** *J. Clin. Oncol.,* 2014, vol. 32, 4066-4072
- **SCHMID, M. et al.** *Advances in experimental medicine and biology,* 2015, vol. 867, 277-89
- **WANG, W. et al.** *Sci. Rep.,* 2014, vol. 4, 5012
- **VLAEMINCK-GUILLEM V. et al.** *Urology.,* 2010, vol. 75, 447-453
- **WADA, Y.** *Methods in molecular biology (Clifton, NJ),* 2013, vol. 951, 245-253
- **TOYAMA, A. et al.** *Anal. Chem.,* 2012, vol. 84, 9655-9662
- **OHYAMA et al.** Glycobiology. Oxford University Press, 2004, vol. 14, 671-679
- **KAMMEIJER et al.** *Analytical Chemistry,* 2018, vol. 90, 4414-4421
- **HAGIWARA et al.** *International Journal of Molecular Sciences,* 2017, vol. 18, 261
- **ISHIKAWA et al.** *International Journal of Molecular Sciences,* 2017, vol. 18, 470